# EUROPEAN PATENT APPLICATION

(11) **EP 3 238 704 A1**
(43) Date of publication of application: **01.11.2017**
(21) Application number: 17172777.9
(22) Date of filing: 28.03.2012
(51) Int. Cl.: A61K 8/81, A61Q 17/04

(54) **SUNCARE COMPOSITIONS AND METHODS**

(30) Priority: 31.03.2011 US 201161470122 P
(62) Divisional of application: 12712050.9
(71) Applicant: Rohm and Haas Company, Philadelphia, PA 19106-2399 (US)
(72) Inventor: WANG, Miao, Collegeville Philadelphia, PA 19426 (US)
(74) Representative: Kent, Venetia Katherine

(57) **Abstract**

Described are suncare compositions comprising (a) one or more fully soluble polymer comprising, as polymerized units, (i) 30% to 75% by weight, based on the weight of said polymer, one or more monomer that has refractive index of 1.490 or higher, (ii) 1% to 30% by weight, based on the weight of said polymer, one or more acid-functional monomer, and (iii) 5% to 69% by weight, based on the weight of said polymer, one or more additional monomer, and (b) at least one suncare active.

## Description

### Field

The present invention relates to personal care compositions, and in particular, suncare compositions.

### Background

One of the most challenging aspects of suncare composition formulation is ensuring that, once applied to a person, the active ingredient remains on the skin after exposure to water or perspiration. On the other hand, the composition must be capable of being removed by conventional soaps or other cleansers. In the past, one strategy was to incorporate film forming ingredients into sunscreen lotions and creams.

However, most of the film formers that are suitable for sunscreen lotion and creams are not compatible for alcohol-based sunscreen spray products, due to solubility/stability issues that will cause spray hardware (for example, the valve and actuator, or pump) to eventually clog. Thus, what is needed is a suncare composition that overcomes these obstacles and performs better than conventional compositions.

### Detailed Description

In one embodiment, the present invention provides suncare compositions comprising (a) one or more fully soluble polymer comprising, as polymerized units, (i) 30% to 75% by weight, based on the weight of said polymer, one or more monomer that has refractive index of 1.490 or higher, (ii) 1% to 30% by weight, based on the weight of said polymer, one or more acid-functional monomer, and (iii) 5% to 69% by weight, based on the weight of said polymer, one or more additional monomer, and (b) at least one suncare active.

"Suncare compositions" relates to compositions to be topically applied to a person (including mouth, ear, and nasal cavities, but not ingested). Such compositions must be cosmetically acceptable, that is, contain ingredients typically used in personal care compositions, and this is intended to underscore that materials that are toxic when present in the amounts typically found in personal care compositions are not contemplated as part of the present invention.

Suncare compositions include lotions, creams and sprays, and are preferably sunscreens, more preferably with an SPF greater than 25. In one embodiment, the suncare composition is an alcohol-based spray. In one embodiment, polymer (a) is alcohol-soluble, showing good solubility in alcohol, and alcohol/water solvents, resulting in a clear solution at use concentration 0.5-7% by weight. In one embodiment, the suncare active enhances solubility and compatibility of polymer (a) in sunscreen spray formulations.

In one embodiment, the suncare active is an organic pigment particulate or an inorganic metal oxide sunscreen particle. "Organic pigment particulate" refers to carbon-based particles of less than about 250 nm, preferably less than about 200 nm. In one embodiment, the organic pigment particulates are bisoctrizole. Bisoctrizole (INCI name: methylene bis-benzotriazolyl tetramethylbutylphenol) is a benzotriazole based organic compound which acts as a broad spectrum UV absorber, effective in both UVA and UVB ranges. Bisoctrizole is available from Ciba Specialty Chemicals under the tradename TINOSORB M. In one embodiment, the organic pigment particulates are present in an amount from about 0.1% to about 50% by weight of the composition, and more preferably 1% to about 25%. In a most preferred embodiment, the organic pigment particulates are present in an amount constituting about 10% active.

Preferably, the inorganic metal oxide sunscreen particles are selected from zinc oxide (ZnO), titanium dioxide (TiO₂), or mixtures thereof. In one embodiment, the inorganic metal oxide sunscreen particles are pigment grade zinc oxide or pigment grade titanium dioxide. In one embodiment, the inorganic metal oxide sunscreen particles are transparent zinc oxide or transparent titanium dioxide. Most inorganic metal oxide sunscreen particles used in a sunscreen produce a cosmetically undesirable white appearance caused by light scattering. Thus, the term "transparent inorganic metal oxide sunscreen particles," as used herein has a special meaning, referring to inorganic metal oxide sunscreen particles produced by a variety of processing conditions which render the inorganic metal oxide compositions as clear, or transparent, upon application. In other words, these specially processed inorganic metal oxide compositions do not appear white once applied, hence the moniker of transparent. Examples of transparent zinc oxide are disclosed in, for example, US Patent Nos. 5,223,250, 5,372,805, 5,573,753, 5,587,148, and 5,876,688. One example of a transparent zinc oxide is commercially available under the tradename Z-COTE from BASF Corporation (Germany). Another example of transparent zinc oxide is commercially available under the tradename ZINCLEAR IM from Antaria Limited (Australia). Another example of transparent zinc oxide is commercially available under the tradename Z-CLEAR from Actifirm (USA). Examples of transparent titanium dioxide are disclosed in, for example, US Patent Nos. 5,573,753, 5,733,895, and 7,390,355. Examples of transparent titanium dioxide are commercially available under the tradenames TIPAQUE and TTO-51(A) from Ishihara Sangyo Kaisha, Ltd. (Japan). Another example of a transparent titanium dioxide is commercially available under the tradename T-COTE from BASF Corporation (Germany). Another example of transparent titanium dioxide is commercially available under the tradename UFTR from Miyoshi Kasei (Japan). Another example of transparent titanium dioxide is commercially available under the tradename SOLA VEIL CLARUS from Uniqema (Great Britain). In one embodiment, the transparent inorganic metal oxide sunscreen particles are selected from transparent zinc oxide, titanium dioxide, or mixtures thereof.

In one embodiment, the suncare active is at least one of octyl methoxycinnamate, avobenzone, para aminobenzoic acid, homosalate, titanium dioxide, zinc oxide, benzophenones, benzylidenes, salicylates, or other known UV filters, including octocrylene, octisalate, and oxybenzone.

One example of a class of monomers that are useful in the present invention are, for example, ethylenically unsaturated monomers (i.e., monomers that have at least one carbon-carbon double bond). Typical ethylenically unsaturated monomers have molecular weight of less than 500. Among such monomers are, for example, vinyl monomers. Some suitable vinyl monomers include, for example, styrene, substituted styrenes, dienes, ethylene, ethylene derivatives, and mixtures thereof. Ethylene derivatives include, for example, unsubstituted or substituted versions of the following: vinyl acetate, acrylonitrile, (meth)acrylic acids, (meth)acrylates, (meth)acrylamides, vinyl chloride, halogenated alkenes, and mixtures thereof. As used herein, "(meth)acrylic" means acrylic or methacrylic; "(meth)acrylate" means acrylate or methacrylate; and "(meth)acrylamide" means acrylamide or methacrylamide. "Substituted" means having at least one attached chemical group such as, for example, alkyl group, alkenyl group, vinyl group, hydroxyl group, carboxylic acid group, other functional groups, and combinations thereof.

In some embodiments, polymer (a) contains one or more vinyl polymer. In some embodiments, every polymer (a) in the suncare composition is a vinyl polymer. As used herein, a vinyl polymer is a polymer made by polymerization of vinyl monomers. In some embodiments, a vinyl polymer is made by free radical polymerization of vinyl monomers.

Independent of the composition of polymer (a), in some embodiments, one or more polymer (a) is used that has Mw of 25,000 or higher, or 50,000 or higher. Independently, in some embodiments, one or more polymer (a) is used that has Mw of 300,000 or lower, or 150,000 or lower. Independently, in some embodiments, every polymer (a) has Mw of 25,000 to 300,000. Polymer molecular weights can be measured by standard methods such as, for example, size exclusion chromatography (SEC, also called gel permeation chromatography or GPC). Generally, polymers have weight-average molecular weight (Mw) of 1,000 or more. Some polymers are characterized by Mn, the number-average molecular weight.

Polymer (a) contains polymerized units of one or more monomer (known herein as "monomer (i)") that has refractive index of 1.490 or higher. Refractive index of a monomer can be measured, for example, by ASTM Standard D1218-02, at 25°C. In some embodiments, monomer (i) contains one or more monomer with refractive index of 1.500 or higher; or 1.530 or higher. In some embodiments, every monomer (i) is a monomer with refractive index of 1.530 or higher.

In some embodiments, monomer (i) contains one or more vinyl monomer. In some embodiments, monomer (i) contains one or more vinyl aromatic monomer. A vinyl aromatic monomer is a monomer that contains one or more carbon-carbon double bond and one or more aromatic ring. Suitable vinyl aromatic monomers include, for example, monomers with benzyl groups, monomers with phenyl groups, styrene, derivatives of styrene (such as, for example, alpha-methyl styrene), and mixtures thereof. In some embodiments, every monomer (i) is a vinyl aromatic monomer. In some embodiments, monomer (i) comprises one or more of styrene, alpha-methyl styrene, or a mixture thereof. In some embodiments, every monomer (i) is selected from styrene, alpha-methyl styrene, and mixtures thereof. Mixtures of suitable monomers (i) are also suitable.

The amount of polymerized units of monomer (i) in the polymer (a) is 30% to 75% by weight, based on the weight of the polymer (a). In some embodiments, the amount of polymerized units of monomer (i) is 35% or more, or 39% or more, by weight, based on the weight of the polymer. In some embodiments, the amount of polymerized units of monomer (i) is 65% or less, or 55% or less, by weight, based on the weight of the polymer. As used herein "weight of polymer" means the dry weight of polymer.

Polymer (a) additionally contains one or more polymerized unit of one or more monomer (herein called "monomer (ii)") that has at least one acid-functional group. Suitable acid-functional groups include, for example, sulfonic acid groups and carboxylic acid groups. The acid-functional groups may be in neutral form or ionic form or a mixture thereof. Some suitable monomer (ii) include, for example, vinyl monomers with at least one acid-functional group. Independently, in some embodiments at least one monomer (ii) with a carboxylic acid group is used. In some embodiments, every monomer (ii) has a carboxylic acid group.

Suitable monomers (ii) having sulfonic acid group include, for example, 2-acrylamido-2-methylpropane sulfonic acid. Suitable monomers (ii) include, for example, acrylic acid, methacrylic acid, and mixtures thereof.

In some embodiments, monomer (ii) comprises at least one monomer that has exactly one acid-functional group. In some embodiments, polymer (a) does not include any polymerized unit of maleic anhydride. In some embodiments, polymer (a) does not include any polymerized unit of any monomer with any anhydride group. In some embodiments, polymer (a) does not include any polymerized unit of any monomer with more than one carboxyl group. In some embodiments, polymer (a) does not include any polymerized unit of any monomer with more than one acid-functional group. Mixtures of suitable monomers (ii) are also suitable.

The amount of polymerized units of monomer (ii) in the polymer is 1% to 30% by weight, based on the weight of the polymer. In some embodiments, the amount of polymerized units of monomer (ii) in the polymer is 2% or more; or 5% or more; or 10% or more; or 12% or more, or 14% or more, or 18% or more, or 20% or more, or 22% or more, by weight, based on the weight of the polymer.

In some embodiments, every monomer (i) that is present is a monomer that has no acid functional group. Independently, in some embodiments, every monomer (ii) that is present is a monomer that has index of refraction below 1.490. Also contemplated are embodiments in which every monomer (i) that is present is a monomer that has no acid functional group and in which every monomer (ii) that is present is a monomer that has index of refraction below 1.490.

Also contemplated are embodiments in which one or more monomer is used that has index of refraction of 1.490 or greater and also has at least one acid functional group. In such embodiments, it is contemplated to calculate the amount of polymerized units of monomers (i) and (ii) in polymer (a) by finding the total weight of polymerized units of monomers that have index of refraction of 1.490 or greater or that have at least one acid functional group or that have both index of refraction of 1.490 and at least one functional group, counting each polymerized unit once. That total weight will be 31 % to 95% by weight, based on the weight of polymer (a).

Among embodiments in which one or more monomer is used that has index of refraction of 1.490 or greater and also has at least one acid functional group, some suitable such monomers are, for example, styrenesulfonic acid and substituted styrene sulfonic acids.

The polymer (a) additionally contains polymerized units of one or more additional monomer (known herein as "monomer (iii)"). Monomer suitable as monomer (iii) is monomer that is not monomer (i) and is not monomer (ii). In some embodiments, monomer (iii) includes one or more vinyl monomer. In some embodiments, every monomer (iii) is a vinyl monomer.

Some suitable monomers (iii) include, for example, olefins, dienes, and (meth)acrylate monomers. As used herein, (meth)acrylate monomers include substituted and unsubstituted esters and amides of acrylic acid and methacrylic acid. Some suitable monomers (iii) include, for example, alkyl esters of (meth)acrylic acid, including, for example, those in which the alkyl group is linear, branched, cyclic, or a combination thereof, with 1 to 20 carbon atoms. In some embodiments, monomer (iii) includes one or more C₁-C₂₀ alkyl acrylate. In some embodiments, monomer (iii) includes one or more alkyl acrylate with 2 or more carbon atoms, or with 3 or more carbon atoms. Independently, in some embodiments, monomer (iii) includes one or more alkyl acrylate with 10 or fewer carbon atoms, or with 8 or fewer carbon atoms. In some embodiments in which one or more alkyl acrylate is used, the amount of polymerized units of alkyl acrylate monomer in the polymer is 5% or more, or 10% or more, by weight based on the weight of the polymer. Independently, in some embodiments in which one or more alkyl acrylate is used, the amount of polymerized units of alkyl acrylate monomer in the polymer is 50% or less, or 40% or less, by weight based on the weight of the polymer.

Independently, in some embodiments, monomer (iii) includes one or more C₁-C₂₀ alkyl methacrylate. In some embodiments, monomer (iii) includes one or more alkyl methacrylate with 6 or fewer carbon atoms, or with 3 or fewer carbon atoms, or with 2 or fewer carbon atoms. In some embodiments, monomer (iii) contains one or more alkyl acrylate and also contains one or more alkyl methacrylate. In some embodiments in which one or more alkyl methacrylate is used, the amount of polymerized units of alkyl methacrylate monomer in the polymer is 3% or more, or 6% or more, by weight based on the weight of the polymer. Independently, in some embodiments in which one or more alkyl methacrylate is used, the amount of polymerized units of alkyl methacrylate monomer in the polymer is 25% or less, or 12% or less, by weight based on the weight of the polymer.

Independently, some suitable monomers (iii) also include, for further example, substituted-alkyl esters of (meth)acrylic acid, which have the structure of alkyl esters of (meth)acrylic acid in which the ester group has one or more substituent group such as, for example, one or more hydroxyl group. Some suitable monomers (iii) include, for example, hydroxyalkyl esters of (meth)acrylic acid in which the alkyl group has 1 to 10 carbon atoms. In some embodiments, monomer (iii) contains one or more hydroxyalkyl ester of (meth)acrylic acid in which the alkyl group has 6 or fewer carbon atoms, or 4 or fewer carbon atoms. Some suitable hydroxyalkyl esters of (meth)acrylic acid include, for example, hydroxypropyl (meth)acrylate, hydroxyethyl (meth)acrylate, and mixtures thereof. In some embodiments in which one or more substituted-alkyl ester of (meth)acrylic acid is used, the amount of polymerized units of substituted-alkyl ester of (meth)acrylic acid in the polymer is 2% or more, or 5% or more, by weight based on the weight of the polymer. Independently, in some embodiments in which one or more substituted-alkyl ester of (meth)acrylic acid is used, the amount of polymerized units of substituted-alkyl ester of (meth)acrylic acid in the polymer is 40% or less, or 20% or less, by weight based on the weight of the polymer.

In some embodiments, monomer (iii) contains one or more alkyl acrylate, one or more alkyl methacrylate, and one or more substituted-alkyl (meth)acrylate. In some embodiments, monomer (iii) does not contain any substituted-alkyl (meth)acrylate. In some embodiments, the sum of the amount polymerized units of monomer (ii) plus the amount polymerized units of hydroxyalkyl esters of (meth)acrylic acid is, by weight based on the weight of the polymer, 10% or more, or 20% or more. Independently, in some embodiments, the sum of the amount of polymerized units of monomer (ii) plus the amount of polymerized units of hydroxyalkyl esters of (meth)acrylic acid is, by weight based on the weight of the polymer, 50% or less, or 40% or less. In some embodiments, the amount of hydroxyalkyl esters of (meth)acrylic acid is 5% or less, or 0%, and the amount of monomer (ii) is 20% or more, by weight based on the weight of the polymer. Independent of the composition of monomer (iii), the total amount in the polymer (a) of polymerized units of all monomer or monomers (iii) is 30% to 89% by weight based on the weight of the polymer (a). In some embodiments, the total amount of polymerized units of monomer (iii) is 75% or less, or 60% or less, by weight based on the weight of the polymer (a). Mixtures of suitable monomers (iii) are also suitable.

In some embodiments, polymer (a) does not include any polymerized unit of any monomer that is a vinyl lactam. Independently, in some embodiments, polymer (a) does not include any polymerized unit of any monomer that is an amide of acrylic acid or an amide of methacrylic acid. Independently, in some embodiments, polymer (a) does not include any polymerized unit of any monomer that is an amide compound. Independently, in some embodiments, polymer (a) does not include any polymerized unit of vinyl acetate. Independently, in some embodiments, polymer (a) does not include any polymerized unit of any monomer that has molecular weight of 500 or greater.

Independently, in some embodiments, all of the polymerized units in the polymer (a) are selected from the group consisting of styrene, alkyl-substituted styrene, alkyl esters of (meth)acrylic acid, hydroxyalkyl esters of (meth)acrylic acid, chain transfer agents, and mixtures thereof.

In one embodiment, a preferred polymer (a) is described in US Patent Application 2009/0252689, the entirety of which is incorporated herein by reference.

In one embodiment, a preferred polymer (a) is ACUDYNE SHINE, available from The Dow Chemical Company.

In one embodiment, a preferred polymer (a) is ACUDYNE BOLD, available from The Dow Chemical Company.

Compositions of the present invention can further incorporate other ingredients known in the art of sunscreen formulations including at least one of cosmetically acceptable emollients, moisturizers, conditioners, oils, suspending agents, opacifiers/pearlizers, surfactants, emulsifiers, preservatives, rheology modifiers, colorants, pH adjustors, propellants, reducing agents, anti-oxidants, fragrances, foaming or de-foaming agents, tanning agents, insect repellants, or biocides. Preferably, sunscreen compositions of the present invention include at least one of a humectant, a surfactant, an emollient, and a preservative. In one embodiment, the sunscreen composition contains decyl glucoside.

In one embodiment, the sunscreen composition contains CORAPAN TQ Diethylhexyl 2,6 Naphthalate or another photostabilizer.

Other optional ingredients for suncare compositions of the present invention include cosmetically acceptable emollients, sunscreens, surfactants, emulsifiers, preservatives, rheology modifiers, colorants, dyes, preservatives, pH adjustors, propellants, reducing agents, fragrances, foaming enhancing agents, tanning agents, astringents, antiseptics, deodorants, antiperspirants, insect repellants, bleaches, lighteners, anti-dandruff agents, or biocides.

Fragrances can be aldehydes, ketones, or oils obtained by extraction of natural substances or synthetically produced as described above. Often, fragrances are accompanied by auxiliary materials, such as fixatives, extenders, stabilizers and solvents.

Biocides include antimicrobials, bactericides, fungicides, algaecides, mildicides, disinfectants, antiseptics, and insecticides.

The amount of optional ingredients effective for achieving the desired property provided by such ingredients can be readily determined by one skilled in the art.

In some embodiments, the suncare composition of the present invention does not contain any silicone compound. Alternatively, in some embodiments, the suncare composition of the present invention does contain at least one silicone compound.

### EXAMPLES

The following examples are for illustrative purposes only and are not intended to limit the scope of the present invention. All percentages are by weight unless otherwise specified.

### Example 1

Exemplary suncare compositions contain the components recited in TABLE 1 on a weight/weight basis (wt. %).

**TABLE 1**

| **Components** | **Formulation A** | **Formulation B** | **Formulation C** |
|---|---|---|---|
| Octinoxate | 7.5 | -- | -- |
| Octisalate | 5 | -- | -- |
| Octocrylene | 0.8 | -- | -- |
| Avobenzone | 1 | -- | -- |
| Oxybenzone | 5 | -- | -- |
| Butyloctyl salicylate | 3.5 | -- | -- |
| Butyl Methoxydibenzoylmethane | -- | 3 | 3 |
| Homos alate | -- | 10 | 10 |
| Ethylhexyl Salicylate | -- | 5 | 5 |
| Benzophenone-3 | -- | 6 | 6 |
| Ethylhexyl Methoxycinnamate | -- | -- | 7.5 |
| PEG-3 Dimethicone | 0-2 | -- | -- |
| PEG-12 Dimethicone | -- | 0-2 | -- |
| PEG-20/PEG-23 Dimethicone | -- | -- | 0-2 |
| Fragrance | as needed | as needed | as needed |
| Polymer (a) described above (Styrene/Acrylates copolymer) | 2 | 2 | 2 |
| SD Alcohol 40B | q.s. to 100 | q.s. to 100 | q.s. to 100 |

Disperse UV absorbers into ethanol solution, and mix at 100-300rpm by overhead mixer until well solubilized. Add silicones, fragrances as needed, then disperse Polymer (a), and mix until clear solution is formed.

### Example 2 - Comparative

Conventional suncare compositions contain the components recited in TABLE 2 on a weight/weight basis (wt. %).

**TABLE 2**

| **Components** | **Comparative Formulation 1** | **Comparative Formulation 2** |
|---|---|---|
| Butyl Methoxydibenzoylmethane | 3 | 3 |
| Homos alate | 10 | 10 |
| Ethylhexyl Salicylate | 5 | 5 |
| Benzophenone-3 | 6 | 6 |
| Ethylhexyl Methoxycinnamate | -- | 7.5 |
| PEG-12 Dimethicone | 0-2 | -- |
| PEG-20/PEG-23 Dimethicone | -- | 0-2 |
| Fragrance | as needed | as needed |
| SD Alcohol 40B | q.s. to 100 | q.s. to 100 |

The composition is prepared substantially according to the protocol of Example 1, with such changes as needed being within the routine knowledge of those skilled in the art.

### Example 3

Compositions made substantially according to the protocols of Examples 1-2 are made and tested for SPF and water resistance properties. Such compositions impart an increase in water resistance, enhance UV actives distribution and/or boost SPF performance.

### Immersion waterproof test protocol:

Samples are immersed in a temperature-controlled water batch (25°C) for 5-80 minutes with constant mixing using a paddle type impeller at 50-150rpm. The volume of the water bath (2L-5L) is large enough to prevent a high concentration of dispersed sunscreen and possibility of re-adsorption. UV spectra are collected per sample in the wavelength range of 240-420nm using LABSPHERE 2000 Spectrometer. Spectra of samples before and after immersion test are compared as measurement of waterproofing performance.

### Example 4

Formulation B, Formulation C, Comparative Formulation 1, and Comparative Formulation 2 are made substantially according to the protocols of Examples 1-2 and tested for contact angle.

### Contact angle / In Vitro Test Method:

Sample preparation: Polymethyl methacrylate plate (Helioplates®) with one face sand-blasted and with a specific roughness (Ra=6-7µm) are loaded with formulation, then a film is cast with a drawn down bar of 10mil thickness. The plate is let to dry under room temperature conditions for 60 minutes.

The wetability of substrate after sunscreen formulation treatment is determined by contact angle measurement. The higher the contact angle, the better water-resistant efficacy. Contact angle measurement is done by using 18 megaohm milliQ water on samples as received approximately 4 seconds after drop placement from a needle. Angles are measured on a Kruss G10 instrument and analyzed using DSA 1.65.0.49 software. Left and right angles are averaged for each individual measurement and a minimum of four drops are measured for each sample. Contact angle results are presented in TABLE 3:

**TABLE 3**

| | **Contact angle** |
|---|---|
| Formulation B | 91.3 |
| Formulation C | 90.7 |
| Comparative Formulation 1 | 80.8 |
| Comparative Formulation 2 | 75.5 |

The inventive formulations display higher contact angles, indicating enhanced water-resistance efficacy.

### Example 5

An exemplary suncare composition and a conventional suncare composition contain the components recited in TABLE 4 on a weight/weight basis (wt. %).

**TABLE 4**

| **Components** | **Formulation D** | **Comparative Formulation 3** |
|---|---|---|
| SD Alcohol 40 | 65.5 | 65.5 |
| Polymer (a) described above (Styrene/Acrylates copolymer) | 2.5 | -- |
| DERMACRYL 79 (Acrylates/Octylacrylamide Copolymer) | -- | 2.5 |
| Octisalate USP | 5 | 5 |
| Avobenzone | 3 | 3 |
| Octocrylene | 3 | 3 |
| Butyl octyl salicylate | 6 | 6 |
| Oxybenzone | 5 | 5 |
| Homos alate | 10 | 10 |

Formulation D and Comparative Formulation 3 are alcohol based sunscreen formulations, made substantially according to the protocols of Examples 1-2 respectively. The formulations are tested for both static SPF and water resistance properties, using the FDA SPF test protocol on at least 5 subjects, modified so that the aerosol can containing the formulation to be tested is shaken and then sprayed into a weigh boat, then 2 mg/cm² is applied to a subject via pipette and finger cot, and allowed to dry for 15 minutes.

Static SPF for Formulation D was 30.42 (Standard deviation of 1.79 +/- 0.8), and Static SPF for Comparative Formulation 3 was 27.51 (Standard deviation of 4.22 +/- 1.72).

For water resistance, after the formulation to be tested is applied as described above and allowed to dry for 15 minutes, then the subject undergoes four cycles of 20 minutes of continuous movement in water separated by 15 minute rests. The percentage difference in retained SPF activity for Formulation D was 34.32% (Standard deviation of 10.26 +/- 4.19), and for Comparative Formulation 3 was 28.25% (Standard deviation of 3.81 +/- 1.71). Accordingly, inventive Formulation D exhibits significantly better water resistance (retention of SPF when contacted by water) as compared to the conventional formulation.

It is understood that the present invention is not limited to the embodiments specifically disclosed and exemplified herein. Various modifications of the invention will be apparent to those skilled in the art. Such changes and modifications may be made without departing from the scope of the appended claims.

Moreover, each recited range includes all combinations and subcombinations of ranges, as well as specific numerals contained therein. Additionally, the disclosures of each patent, patent application, and publication cited or described in this document are hereby incorporated herein by reference, in their entireties.

## Claims

1. A suncare composition comprising
(a) one or more fully soluble polymer comprising, as polymerized units,
(i) 30% to 75% by weight, based on the weight of said polymer, one or more monomer that has refractive index of 1.490 or higher,
(ii) 1% to 30% by weight, based on the weight of said polymer, one or more acid-functional monomer, and
(iii) 5% to 69% by weight, based on the weight of said polymer, one or more additional monomer, and
(b) at least one suncare active.

2. The suncare composition of claim 1, wherein the suncare active is at least one of octyl methoxycinnamate, octocrylene, octisalate, oxybenzone, avobenzone, para aminobenzoic acid, homosalate, titanium dioxide, zinc oxide, benzophenones, benzylidenes, or salicylates.

3. The suncare composition of claim 1, wherein the suncare active is a mixture of at least two suncare actives.

4. The suncare composition of claim 1, wherein the composition has a contact angle greater than 87, preferably.

5. The suncare composition of claim 1, wherein the composition is alcohol based and polymer (a) is soluble in alcohol.

6. The suncare composition of claim 1, wherein monomer (i) comprises one or more vinyl aromatic monomer.

7. The suncare composition of claim 1, wherein monomer (iii) comprises one or more hydroxyalkyl (meth)acrylate.

8. The suncare composition of claim 1, further comprising at least one silicone.
